(19) Europäisches Patentamt
European Patent Office
Office européen des brevets

(11) **EP 2 185 216 B1**

(12) **EUROPÄISCHE PATENTSCHRIFT**

(45) Veröffentlichungstag und Bekanntmachung des Hinweises auf die Patenterteilung:
**20.03.2013 Patentblatt 2013/12**

(21) Anmeldenummer: **08785230.7**

(22) Anmeldetag: **30.07.2008**

(51) Int Cl.:
***A61M 1/14*** (2006.01)

(86) Internationale Anmeldenummer:
**PCT/EP2008/006285**

(87) Internationale Veröffentlichungsnummer:
**WO 2009/015882 (05.02.2009 Gazette 2009/06)**

(54) **Dialysierflüssigkeitskreislauf, Dialysegerät mit Dialysierflüssigkeitskreislauf, Verfahren zur Erkennung von Luft in einer einen Dialysierflüssigkeitskreislauf durchströmenden Dialysierflüssigkeit sowie Verwendung eines Gassensors in einem Dialysierflüssigkeitskreislauf**

Dialysis liquid circuit, dialysis apparatus comprising a dialysis liquid circuit, method for detecting air in a dialysis liquid flowing through a dialysis liquid circuit, and use of a gas sensor in a dialysis liquid circuit

Circuit de liquide de dialyse, dialyseur comprenant un circuit de liquide de dialyse, procédé de détection d'air dans un liquide de dialyse circulant dans un circuit de liquide de dialyse et utilisation d'un capteur de gaz dans un circuit de liquide de dialyse

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.07.2007 DE 102007035815**
**22.01.2008 DE 102008005516**

(43) Veröffentlichungstag der Anmeldung:
**19.05.2010 Patentblatt 2010/20**

(73) Patentinhaber: **Fresenius Medical Care Deutschland GmbH**
**61352 Bad Homburg (DE)**

(72) Erfinder:
• **MAIERHOFER, Andreas**
**97422 Schweinfurt (DE)**
• **GAGEL, Alfred**
**96123 Litzendorf (DE)**
• **GROSS, Malte**
**97464 Niederwerrn (DE)**

(74) Vertreter: **Herrmann, Uwe et al**
**Lorenz - Seidler - Gossel**
**Widenmayerstrasse 23**
**80538 München (DE)**

(56) Entgegenhaltungen:
**EP-A- 0 643 301      WO-A-96/04401**
**WO-A-98/11430      DE-A1- 1 964 735**
**US-A- 4 085 046      US-A- 5 783 072**

**Beschreibung**

Die vorliegende Erfindung betrifft einen Dialysierflüssigkeitskreislauf mit Leitungen zur Führung von Dialysierflüssigkeit sowie mit Mitteln zur Erkennung von Luft in der Dialysierflüssigkeit.

Die Anwesenheit von Luft in einer in einem Dialysierflüssigkeitskreislauf geführten Dialysierflüssigkeit ist in mehrfacher Hinsicht unerwünscht. Zum einen können Luftblasen die Messung der Konzentration von Inhaltsstoffen der Dialysierflüssigkeit behindern, wie dies aus der WO 96/04401 bekannt ist. Gemäß dieser Druckschrift wird daher vorgeschlagen, die Dialysierflüssigkeit vor der Konzentrationsmessung zu entgasen. Zum anderen sind Luftblasen für die korrekte Bilanzierung der in den Dialysator eintretenden und aus diesem austretenden Dialysierflüssigkeiten nachteilig, da die gängigen Bilanzeinrichtungen von einer entgasten Dialysierflüssigkeit ausgehen.

Durch Pegelmessung in der Luftabscheidekammer ist es weiterhin bekannt, große Luftmengen im System zu erkennen. Eine schnelle und zuverlässige Möglichkeit zur Überwachung des Dialysierflüssigkeitskreislaufs auf Undichtigkeiten ist damit jedoch nicht gegeben.

Um Undichtigkeiten zu detektieren, sind Lekkagesensoren bekannt, die jedoch nur dann Einsatz finden können, wenn es zum Austreten von Flüssigkeit kommt. Aus dem Stand der Technik ist es ferner bekannt, Undichtigkeiten im Unterdruckbereich des Dialysierflüssigkeitskreislaufes zu detektieren. Dazu wird nach Aufbau eines Unterdrucks der Flüssigkeitskreislauf angehalten, die zu untersuchende Stelle abgedichtet und der zeitliche Druckverlauf gemessen. Vermindert sich der Unterdruck, deutet dies auf das Eindringen von Luft in den Dialysierflüssigkeitskreislauf und damit auf eine Lekkage hin. Nach diesem bekannten Verfahren sind Drucksensoren sowie Ventile zur Abdichtung und Eingrenzung des zu untersuchenden Bereiches notwendig. Ein weiterer Nachteil ergibt sich daraus, dass die Flüssigkeitszirkulation zur Prüfung auf Undichtigkeiten angehalten werden muss.

US 5, 783, 072 offenbart die Durch Führung eines Druckhalteters vor Beginn einer Dialyse-behandlung, bei dem der Flüssigkeitskreislauf unter Druck gesetzt wird.

Der vorliegenden Erfindung liegt die Aufgabe zugrunde, einen Dialysierflüssigkeitskreislauf der eingangs genannten Art dahingehend weiterzubilden, dass mit einem vergleichsweise geringen apparativen Aufwand zuverlässig und bequem Undichtigkeiten im Unterdruckbereich des Dialysierflüssigkeitskreislaufes erfaßt werden können.

Diese Aufgabe wird durch einen Dialysierflüssigkeitskreislauf mit den Merkmalen des Anspruchs 1 sowie durch ein Verfahren gemäß Anspruch 13 gelöst.

Danach ist vorgesehen, dass die Mittel zur Erkennung von Luft in der Dialysierflüssigkeit wenigstens einen kontinuierlich von der Dialysierflüssigkeit durchströmten Gassensor umfassen, der derart ausgeführt ist, dass er wenigstens eine Eigenschaft der Dialysierflüssigkeit mißt, die von der Anwesenheit von Luftblasen in der Dialysierflüssigkeit abhängt und der stromabwärts eines zu überwachenden Bereiches des Dialysierflüssigkeitskreislaufes angeordnet ist, in dem im Betrieb des Dialysierflüssigkeitskreislaufes gegenüber Atmosphärendruck Unterdruck herrscht. Die Mittel weisen des Weiteren eine Auswerteeinheit auf, die mit dem Gassensor in Verbindung steht und die derart ausgeführt ist, dass die mittels des Gassensors gemessene Eigenschaft im Hinblick auf das Vorhandensein von Luftblasen in der Dialysierflüssigkeit ausgewertet wird.

Die Erfindung basiert somit auf dem Einsatz eines im Betrieb des Dialysierflüssigkeitskreislaufes von der Dialysierflüssigkeit durchströmten Gassensors, der stromabwärts des zu untersuchenden Unterdruckbereiches angeordnet ist. Das Signal des Gassensors wird an eine Auswerteeinheit übergeben, die eine Signalauswertung vornimmt. Denkbar ist es beispielsweise, dass die Auswerteeinheit bei Feststellen von Undichtigkeiten aufgrund des Sensorsignals ein Alarmsignal abgibt oder bestimmte Betriebsweisen des Dialysierflüssigkeitskreislaufes veranlasst. Erfindungsgemäß ist damit eine kontinuierliche Erkennung von Luftblasen in der Dialysierflüssigkeit bzw. eine kontinuierliche Überwachung des Dialysierflüssigkeitskreislaufes auf Undichtigkeiten möglich, so dass anders als im Stand der Technik Fehler schnell und zuverlässig erkannt werden.

Gemäß der Erfindung ist vorgesehen, dass der Gassensor stromabwärts eines in dem Dialysierflüssigkeitskreislauf befindlichen Dialysators angeordnet ist. Vorzugsweise befindet sich der Gassensor stromaufwärts einer Pumpe, die die Dialysierflüssigkeit fördert und die die Unterdruckerzeugung bewirkt.

Bei dem Gassensor kann es sich beispielsweise um einen Leitfähigkeitssensor handeln. Solche Leitfähigkeitssensoren werden dabei seit langem in Dialysierflüssigkeitskreisläufen eingesetzt, um die Zusammensetzung der Dialysierflüssigkeit zu bestimmen. Erfindungsgemäß können diese seit langem bekannten Sensoren nun zusätzlich dazu eingesetzt werden, als Gassensor Luftblasen im Dialysierflüssigkeitskreislauf zu detektieren. Hierdurch kann mit minimalem zusätzlichen Aufwand eine effektive Überwachung des Dialysierflüssigkeitskreislaufs auf Undichtigkeiten erfolgen.

Die Leitfähigkeit einer Flüssigkeit kann somit mittels eines Leitfähigkeitssensors bestimmt werden, wobei beispielsweise eine Durchfluß-Messzelle verwendet wird. Im Prinzip erfolgt die Bestimmung der Leitfähigkeit $\sigma$ eines homogenen Mediums durch Messung des elektrischen Widerstandes R zwischen zwei Kontakten im Abstand I, die durch das leitende Medium mit einer mittleren Querschnittfläche A miteinander verbunden sind, gemäß folgender Beziehung:

$$R = 1/\sigma \ l/A \quad \text{und} \quad \sigma = 1/R \ l/A.$$

Da Luft ein Isolator ist, verringert die Anwesenheit von Luftblasen die zur Leitung beitragende mittlere Querschnittsfläche A in der Messstrecke, was zur Folge hat, dass ein größerer elektrischer Widerstand R und damit eine geringere Leitfähigkeit $\sigma$ festgestellt wird.

Bei einem solchen Leitfähigkeitssensor befinden die Kontaktelektoden so in einem Leitungsteil, dass sie in direktem Kontakt mit der Dialysierflüssigkeit stehen. Dabei wir die Leitfähigkeit vorteilhafterweise über die Stärke eines Wechselstroms gemessen, welcher über die Kontaktelektroden durch die Dialysierflüssigkeit fließt.

Außer Leitfähigkeitssensoren sind im Rahmen der Erfindung auch andere Sensoren einsetzbar, die eine Eigenschaft der Dialysierflüssigkeit messen, die durch die Anwesenheit von Luftblasen verändert wird. Hierzu gehören beispielsweise optische Sensoren. Die Verwendung eines kapazitiven Sensors zur Erkennung von Luftblasen ist dabei aus der DE 196 51 355 A1 bekannt.

Denkbar ist, dass je nach Geometrie und Messverfahren sich die Signalstärke bei Anwesenheit von Luft erhöhen kann, wie dies beispielsweise bei Absorptionsmessungen der Fall ist, bei denen die Absorption durch Wasser erfolgt. Denkbar ist ebenfalls, dass sich die Signalstärke bei Anwesenheit von Luft verringert, wie dies beispielsweise der Fall ist, wenn das Licht bei Anwesenheit von Luftblasen stärker gestreut oder auf dem Weg zum Detektor abgelenkt wird. Da Luftblasen sich im allgemeinen nicht homogen in der Luftflüssigkeit verteilen, ist das Auftreten von Signalspitzen charakteristisch für das Vorhandensein von Luftblasen.

Selbstverständlich kann auch ein gemittelter Wert, beispielsweise eine mittlere Leitfähigkeit herangezogen werden, um auf die Anwesenheit von Luftblasen zu schließen.

Dementsprechend kann vorgesehen sein, dass der Gassensor derart ausgeführt ist, dass mittels des Gassensors einzelne Luftblasen in der Dialysierflüssigkeit erfassbar sind. In diesem Fall ist es erforderlich, dass die Messung kürzere Zeiten auflösen kann als die Verweildauer einer Luftblase im Volumen des Gassensors. Unter der Annahme, dass sich die Luftblase mit der gleichen Geschwindigkeit wie die Dialysierflüssigkeit bewegt, bestimmt somit die Zeitauflösung des Gassensors, d. h. beispielsweise die Zeitauflösung der Leitfähigkeitsmesszelle die Reaktion auf die Anwesenheit von Luftblasen. Wird das Sensorsignal in Intervallen t abgetastet, die kürzer sind als die Verweildauer T einer Luftblase im Volumen V des Sensors, bewirkt der Durchtritt einer Luftblase durch die Sensorzelle einen scharfen Einbruch im gemessenen Signal. Dabei gilt

$$T = V/Q, \quad t < T,$$

wobei Q die Flussrate durch die Sensorzelle ist. Typische, die Erfindung jedoch nicht beschränkende Werte in einem Dialysegerät bzw. Dialysierflüssigkeitskreislauf sind V = 2 ml, Q = 500 ml / min, woraus sich eine Verweildauer von T = 0,2 s ergibt.

Denkbar ist es ebenfalls, dass der Gassensor und/oder die Auswerteeinheit derart ausgeführt ist, dass ein Mittelwert der wenigstens einen Eigenschaft der Dialysierflüssigkeit erfaßt wird und der Auswertung zugrunde gelegt wird. Erfolgt eine Mittelung des Sensorsignals über einen Zeitraum t > T, so sind kurzzeitige Änderungen der gemessenen Eigenschaften praktisch nicht mehr erkennbar. Dafür liegt bei Anwesenheit von Luftblasen ein Mittelwert der gemessenen Eigenschaft, beispielsweise der Leitfähigkeit vor, der von dem abweicht, der bei der gleichen Flüssigkeit in Abwesenheit von Luftblasen gemessen würde.

In weiterer Ausgestaltung der Erfindung ist vorgesehen, dass die Auswerteeinheit derart ausgeführt ist, dass sie ein Signal abgibt und/oder eine bestimmte Betriebsweise des Dialysierflüssigkeitskreislaufes oder eines Dialysegerätes, das den Dialysierflüssigkeitskreislauf aufweist, veranlasst, wenn bei der Auswertung des die gemessene Eigenschaft repräsentierenden Signals des Gassensors festgestellt wird, dass Luftblasen in der Dialysierflüssigkeit existieren.

Denkbar ist es beispielsweise, dass ein Druckhaltetest des Dialysierflüssigkeitskreislaufes ausgelöst wird. Werden also aufgrund der kontinuierlichen Messung durch das erfindungsgemäße Verfahren Luftblasen in der Dialysierflüssigkeit entdeckt, kann das System angehalten und eine Nachmessung über den Druckhaltetest erfolgen, z.B. zur Überprüfung, um unnötige Warnmeldungen zu vermeiden, oder um den Fehler zu analysieren. Ein solcher Druckhaltetest kann als Nachmessung, bei welcher ein Testvolumen gezielt bestimmten Druckverhältnissen ausgesetzt wird und der Druckverlauf über die Zeit erfasst wird, effektiv mit der kontinuierlichen Überwachung kombiniert werden, um ein ebenso zuverlässiges wie schnelles Detektieren von Undichtigkeiten zu gewährleisten. Dabei kann für den Druckhaltetest z.B. der Flüssigkeitskreislauf angehalten, die zu untersuchende Stelle abgedichtet und der zeitliche Druckverlauf gemessen werden. Vermindert sich der Unterdruck, deutet dies auf das Eindringen von Luft in den Dialysierflüssigkeitskreislauf und damit auf eine Leckage hin. Hierzu sind bei diesem an sich bekannten Verfahren Drucksensoren sowie Ventile zur Abdichtung und Eingrenzung des zu untersuchenden Bereiches vorgesehen.

Weiterhin wird gegebenenfalls eine Warnung an den Benutzer ausgegeben, wenn festgestellt wird, dass Luftblasen in der Dialysierflüssigkeit vorliegen.

In weiterer Ausgestaltung der Erfindung ist eine Bypassleitung vorgesehen, die den zu überwachenden Bereich umgeht, wobei wenigstens ein Ventil vorgesehen ist, mittels dessen bei geöffnetem Ventil die Durchströmung der Bypassleitung mit Dialysierflüssigkeit zur Durchführung einer Referenzmessung möglich ist. Um die Eigenschaft der Dialysierflüssigkeit in Abwesenheit von Luftblasen bestimmen zu können, wird diese mittels der Bypassleitung um den zu überwachenden Bereich herumgeführt und eine Eigenschaft der Dialysierflüssigkeit gemessen. Auf der Grundlage dieses Referenzwertes kann sodann eine Bestimmung dahingehend vorgenommen werden, ob nach der Durchströmung des zu überwachenden Bereiches mit Dialysierflüssigkeit Luftblasen in der Dialysierflüssigkeit vorliegen.

In weiterer Ausgestaltung der Erfindung ist vorgesehen, dass stromaufwärts des zu überwachenden Bereiches des Dialysierflüssigkeitskreislaufes ein weiterer Gassensor zur Durchführung einer Referenzmessung angeordnet ist. Somit kann vor dem zu überwachenden Bereich mit einem zweiten Gassensor gemessen werden, welche Eigenschaft, z. B. welche Leitfähigkeit die luftblasenfreie Dialysierflüssigkeit aufweist. Ist die Eigenschaft nach dem Durchströmen des zu überwachenden Bereiches gegenüber der der luftfreien Dialysierflüssigkeit verändert, so deutet dies auf einen Lufteintrag , d. h. auf eine Leckage hin. Liegt eine Verminderung z. B. der Leitfähigkeit gegenüber der genannten Referenzmessung vor, kann daraus nicht nur auf das Vorhandensein von Luft, sondern auch auf die Menge der eintretenden Luft geschlossen werden.

In weiterer Ausgestaltung der Erfindung ist vorgesehen, dass Mittel zur Veränderung des Druckes in dem zu überwachenden Bereich angeordnet sind. Eine etwaige Leckage lässt sich durch Variation des Unterdruckes in dem zu überwachenden Bereich erkennen. Da bei einem Leck die Eindringrate der Luft bei steigendem Druckgradienten steigt, führt eine Absenkung des Innendrucks zu einem verstärkten Eindringen von Luft. Wird die Absenkung des Innendrucks durch Flusserhöhung einer stromabwärts des Unterdruckbereichs liegenden Förderpumpe hervorgerufen, kann dies dazu führen, dass die Menge der einströmenden Luft zwar absolut zunimmt, deren Anteil im Verhältnis zum Gesamtstrom jedoch konstant bleibt, so dass eine Änderung der gemessenen Eigenschaft ausbleibt. Besonders vorteilhaft ist es daher, wenn Innendruck und Volumenstrom entkoppelt sind. Dazu kann vorgesehen sein, dass stromaufwärts des zu überwachenden Bereiches ein Druckregler angeordnet ist, der beispielsweise durch ein regelbares Ventil oder auch durch eine regelbare Pumpe gebildet wird.

In weiterer Ausgestaltung der Erfindung ist vorgesehen, dass in dem Dialysierflüssigkeitskreislauf wenigstens ein Drucksensor zur Erfassung des in der Dialysierflüssigkeit herrschenden Druckes angeordnet ist. Grundsätzlich existieren unterschiedliche Entstehungsmöglichkeiten für Luftblasen bzw. Gasblasen innerhalb der Dialysierflüssigkeit. Eine Möglichkeit ist der oben erwähnte Lufteintritt aufgrund einer Leckage und eine andere Möglichkeit ist eine Nachentgasung der Dialysierflüssigkeit. Mittels eines Drucksensors, vorzugsweise in der Nachbarschaft des Gassensors, kann zwischen beiden Entstehungsmöglichkeiten für Gasblasen unterschieden werden. Liegt der Druck oberhalb eines zur massiven Nachentgasung nötigen Unterdrucks (> - 350 mmHg), so müssen die Gasblasen durch externen Lufteintritt entstanden sein. Liegt hingegen ein starker Unterdruck vor, so können Gasblasen durch beide Möglichkeiten (Leckage, Nachentgasung) entstanden sein.

Die genannte Nachentgasung der Dialysierflüssigkeit tritt vor allem dann auf, wenn die primärseitige Entgasung ungenügend war oder im Dialysat durch einen hohen Bicarbonatgehalt ein vergleichsweise hoher $CO_2$- Partialdruck vorliegt.

Im Gegensatz zu einer Luftdetektion allein durch das Anspringen einer Sekundärluftabscheidung, wie sie aus dem Stand der Technik bekannt ist, hat die Detektion gemäß der vorliegenden Erfindung den Vorteil, dass hier Luftblasen sofort erkannt werden und nicht erst auf eine Akkumulation in einem Sekundärabscheider gewartet werden muss.

Nach der bisherigen Lösung, bei der stromabwärts des Dialysators Sekundärluft abgeschieden wurde, findet keine Unterscheidung zwischen Lufteintrag und Nachentgasung statt. Die bisher bekannte Lösung sieht vor, dass für den Fall, dass ein Absinken des Flüssigkeitsspiegels in der Luftabscheidekammer detektiert wird, die Luft durch das Öffnen eines Ventils oder durch eine Pumpe aus der Kammer entfernt wird.

Die vorliegende Erfindung betrifft des weiteren ein Verfahren zur Erkennung von Luft in einer einen Dialysierflüssigkeitskreislauf durchströmenden Dialysierflüssigkeit, bei dem stromabwärts eines zu überwachenden Bereiches des Dialysierflüssigkeitskreislaufes, in dem im Betrieb des Dialysierflüssigkeitskreislaufes gegenüber Atmosphärendruck Unterdruck herrscht, kontinuierlich eine Eigenschaft der einen Messbereich, d. h. einen Bereich beispielsweise unmittelbar neben einem Gassensor, durchströmenden Dialysierflüssigkeit gemessen wird, die von der Anwesenheit von Luftblasen in der Dialysierflüssigkeit abhängt, wobei die gemessene Eigenschaft oder ein darauf basierendes Signal im Hinblick auf das Vorhandensein von Luftblasen in der Dialysierflüssigkeit ausgewertet wird. Insbesondere handelt es sich dabei vorteilhafterweise um ein Verfahren zur Überwachung des Dialysierflüssigkeitskreislaufs auf Undichtigkeiten.

Vorzugsweise handelt es sich bei der gemessenen Eigenschaft um die Leitfähigkeit der Dialysierflüssigkeit.

In weiterer Ausgestaltung der Erfindung ist vorgesehen, dass einzelne Signalspitzen der Eigenschaft, beispielsweise einzelne Signalspitzen der Leitfähigkeit, gemessen werden und dass auf dieser Grundlage eine Auswertung erfolgt. Alternativ dazu ist auch denkbar, dass eine Mittelwertbildung der Eigenschaft erfolgt und dass auf der Grundlage des Mittelwertes die Auswertung vorgenommen wird.

Wie oben ausgeführt, kann vorgesehen sein, dass für den Fall, dass Luftblasen in der Dialysierflüssigkeit ermittelt werden, ein Signal abgegeben und/oder eine bestimmte Betriebsweise des Dialysierflüssigkeitskreislaufes veranlasst wird.

Dabei kann es sich z. B. um einen Druckhaltetest des Dialysierflüssigkeitskreislaufs handeln. Dieser kann, wie oben beschrieben, als Nachmessung zur Überprüfung eingesetzt werden. Auch andere Betriebsweisen, wie etwa der Abbruch der Dialysebehandlung und/oder die Ausgabe einer entsprechenden Warnung sind denkbar.

Wie oben ausgeführt, kann eine Referenzmessung durchgeführt werden, mittels derer die fragliche Eigenschaft, beispielsweise die Leitfähigkeit der luftblasenfreien Dialysierflüssigkeit ermittelt wird.

Die Referenzmessung kann dadurch vorgenommen werden, dass Dialysierflüssigkeit im Bypass um den zu überwachenden Bereich geführt wird. Sie kann auch dadurch vorgenommen werden, dass die Eigenschaft der Dialysierflüssigkeit stromaufwärts des zu überwachenden Bereiches gemessen wird.

Soll keine Referenzmessung durchgeführt werden oder ist keine Referenzmessung möglich, kann auf Luftblasen auch dadurch geschlossen werden, dass der Druck in dem zu überwachenden Bereich variiert wird, wobei vorzugsweise vorgesehen ist, dass dabei die Strömungsrate der Dialysierflüssigkeit nicht verändert wird.

Die vorliegende Erfindung betrifft, gemäß Anspruch 24, des Weiteren die Verwendung eines Gassensors in einem Dialysierflüssigkeitskreislauf, die dadurch gekennzeichnet ist, dass der Gassensor zur Erkennung von Luftblasen in einer einen Dialysierflüssigkeitskreislauf durchströmenden Dialysierflüssigkeit dient und derart ausgeführt ist, dass er wenigstens eine Eigenschaft der Dialysierflüssigkeit mißt, die von der Anwesenheit von Luftblasen in der Dialysierflüssigkeit abhängt, wobei der Gassensor stromabwärts eines zu überwachenden Bereiches des Dialysierflüssigkeitskreislaufes angeordnet ist, in dem im Betrieb des Dialysierflüssigkeitskreislaufes gegenüber Atmosphärendruck Unterdruck herrscht, und kontinuierlich von der Dialysierflüssigkeit durchströmt wird. Insbesondere handelt es sich dabei vorteilhafterweise um die Verwendung des Gassensors zur Überwachung des Dialysierflüssigkeitskreislaufs auf Undichtigkeiten.

Bevorzugte Ausgestaltungen der erfindungsgemäßen Verwendung eines Gassensors sind Gegenstand der Unteransprüche 25 bis 34.

Die Erfindung betrifft schließlich ein Dialysegerät mit einem Dialysierflüssig-keitskreislauf gemäß einem der Ansprüche 1 bis 12.

Bei der Dialysierflüssigkeit, welche erfindungsgemäß auf Luftblasen überwacht wird, kann es sich dabei um jede Flüssigkeit handeln, welche in einer Betriebsphase eines Dialysegeräts durch den Dialysierflüssigkeitskreislauf strömt, wie zum Beispiel reines Ultrafiltrat oder auch Reinigungsflüssigkeit. Auch in diesen Betriebsphasen kann so der Dialysierflüssigkeitskreislauf erfindungsgemäß auf Undichtigkeiten überwacht werden.

Weitere Einzelheiten und Vorteile der Erfindung werden anhand eines in der Zeichnung dargestellten Ausführungsbeispiels näher erläutert. Es zeigen:

Figur 1: eine schematische Darstellung einer Anordnung zur Leckerkennung durch Leitfähigkeitsmessung mittels Signalspitzen-Detektion,

Figur 2: eine schematische Darstellung einer Anordnung zur Leckerkennung durch Ermittlung der gemittelten Leitfähigkeit mittels Bypassleitung oder zweitem Sensor

Figur 3: eine schematische Darstellung einer Anordnung zur Leckerkennung durch Ermittlung der gemittelten Leitfähigkeit mittels Druckänderung und

Figur 4: die Darstellung des Leitfähigkeitsverlaufs über die Zeit stromaufwärts und stromabwärts eines Dialysators.

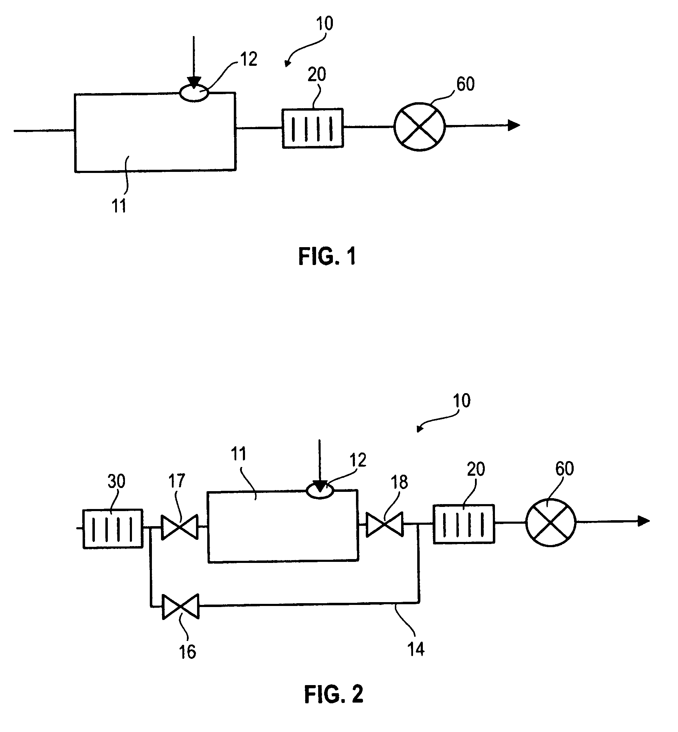

Figur 1 zeigt eine schematische Darstellung der Komponenten des Dialysierflüssigkeitskreislaufes gemäß der Erfindung, wobei die Erkennung auf Undichtigkeiten im Unterdruckbereich des Dialysierflüssigkeitskreislaufes durch Signalspitzen-Detektion erfolgt.

Mit dem Bezugszeichen 10 ist der Dialysierflüssigkeitskreislauf gekennzeichnet, dessen Bereich 11 den auf Undichtigkeiten zu überwachenden Bereich des Dialysierflüssigkeitskreislaufes darstellt. Stromabwärts des zu überwachenden Bereiches 11 befindet sich der Gassensor 20, der in dem hier dargestellten Beispiel als Leitfähigkeitssensor ausgeführt ist. Insbesondere kann dabei auf einen oftmals bereits vorhandenen Leitfähigkeitssensor zurückgegriffen werden, welcher nun zusätzlich zum erfindungsgemäßen Erkennen von Luftbläschen herangezogen wird.

Das Bezugszeichen 60 kennzeichnet eine stromabwärts des zu überwachenden Bereiches 11 und stromabwärts des Gassensors 20 angeordnete Pumpe. Im Betrieb der Pumpe befindet sich der zu überwachende Bereich 11 auf der Pumpensaugseite, so dass in diesem ein Unterdruck vorliegt.

Das Bezugszeichen 12 kennzeichnet eine Leckstelle des zu überwachenden Bereiches 11, durch die Luft in den zu überwachenden Bereich 11 eintritt, wie dies durch den Pfeil angedeutet ist. Diese in der den zu überwachenden Bereich 11 durchströmenden Dialysierflüssigkeit Luftblasen bildende Luft kann nun beispielsweise durch Signalspitzen-Detektion erfasst werden.

Wie oben ausgeführt, muss hierzu das Signal des Gassensors 20 mit einer Abtastrate aufgenommen werden, die mit der Verweildauer einer Luftblase in dem Sensor 20 vergleichbar ist. Luftblasen können dann detektiert werden, wenn sie Leitfähigkeitseinbrüche verursachen, die größer sind als das Rauschen der Leitfähigkeitsmessung. Je höher die Leitfähigkeit der Flüssigkeit ist, umso größer ist der Effekt von Luftblasen, die wie oben ausgeführt, die Leitfähigkeit des Luft-Dialysierflüssigkeitsgemisches verringern. Unterliegt die Leitfähigkeit in dem zu überwachenden Bereich 11 Schwankungen, z. B. aufgrund von Schwankungen im Mischsystem des Dialysegerätes, so können luftinduzierte Signalspitzen von Leitfähigkeitsschwankungen der luftfreien Flüssigkeit dadurch unterschieden werden, dass letztere deutlich langsamer erfolgen. Eine Temperaturkompensation der Leitfähigkeit ist im allgemeinen nicht nötig, da die luftinduzierten Signalspitzen vergleichsweise kurz sind und da sich die Temperaturschwankungen langsamer auf die Leitfähigkeit auswirken.

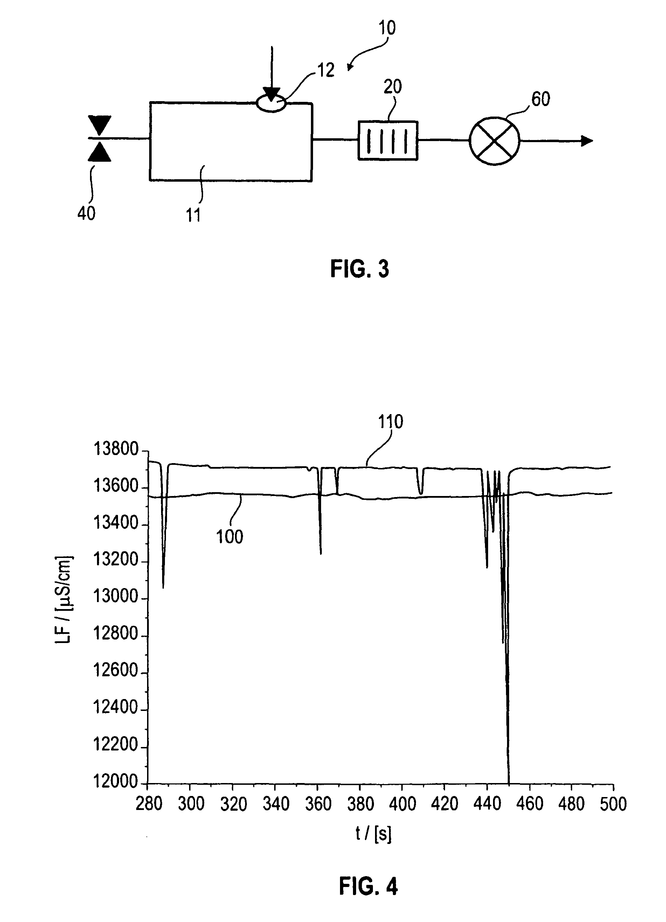

Bei der aus Figur 1 ersichtlichen Anordnung ist es möglich, eine Signalspitzen-Detektion durchzuführen, bei der die Leitfähigkeit über die Zeit erfaßt wird und bei der Signalspitzen durch kurz andauernde Leitfähigkeitsverringerungen aufgrund von Luftblasen auftreten. Dies ist beispielsweise aus Figur 4 ersichtlich, in der zu unterschiedlichen Zeitpunkten auftretenden Signalspitzen dargestellt sind. Die in etwa horizontal verlaufende Linie 100 zeigt die Leitfähigkeit stromaufwärts des zu überwachenden Bereiches beispielsweise stromaufwärts eines Dialysators und die die Signalspitzen aufweisende Linie 110 zeigt die Leitfähigkeit stromabwärts des zu überwachenden Bereiches.

Sollte eine Quantifizierung des Lufteintrages mit der Anordnung gemäß Figur 1 erforderlich sein, setzt dies einen groben Abgleich der Leitfähigkeitszelle voraus, so dass aufgrund der Anzahl bzw. Höhe der Signalspitzen auf die Anzahl bzw. Menge der Luftblasen geschlossen werden kann.

Soll ein besseres Signal/Rauschverhältnis erreicht werden, kann das Leitfähigkeitssignal des Gassensors über einen längeren Zeitraum gemittelt werden. Als Folge der längeren Mittlungszeit kann es dazu kommen, dass die luftinduzierten Leitfähigkeitssignalspitzen nicht mehr sichtbar sind, sondern dass nur noch eine Verringerung des gemittelten Leitfähigkeitswertes gegenüber einer luftblasenfreien Dialysierflüssigkeit vorliegt. Bei diesem Messverfahren ist eine Kompensation von Temperatureinflüssen bei der Messung der betreffenden Eigenschaft, d. h. z. B. bei der Leitfähigkeitsmessung, anzustreben sowie auch eine gute thermische Isolation des Gassensors gegenüber Umgebungseinflüssen.

Eine Luftblasendetektion erfolgt durch abwechselnde Messung der Leitfähigkeit in einem Zustand, in dem mit Sicherheit keine Luft in der Dialysierflüssigkeit vorliegt, und in einem Zustand, in dem der Eintrag von Luft im System möglich ist.

Figur 2 zeigt eine derartige Anordnung zur Lek-

kageerkennung gemäß diesem Prinzip, wobei gleiche oder funktionsgleiche Komponenten mit denselben Bezugszeichen gekennzeichnet sind wie in Figur 1.

Zunächst wird die Leitfähigkeit der zirkulierenden Dialysierflüssigkeit in Abwesenheit von Luftblasen bestimmt, z. B. durch Messung mittels des Leitfähigkeitssensors 20 unter Umgehung des zu überwachenden Bereiches 11 mittels der Bypassleitung 14. In dieser ist das Ventil 16 angeordnet, mittels dessen die Bypassleitung 14 verschlossen oder geöffnet werden kann. Für die Referenzmessung wird das Ventil 16 geöffnet und die Ventile 17, 18, die sich stromaufwärts und stromabwärts des zu überwachenden Bereiches 11 befinden, werden geschlossen. Liegt der Leitfähigkeitsmesswert der luftblasenfreien Dialysierflüssigkeit vor, wird das Ventil 16 geschlossen und es erfolgt die Durchströmung des zu überwachenden Bereiches 11 bei geöffneten Ventilen 17, 18. Liegt der mittels des Sensors 20 gemessene Leitfähigkeitswert sodann unter dem der Referenzmessung, liegt ein Lufteintrag über die Leckstelle 12 vor.

Alternativ dazu kann die Referenzmessung auch kontinuierlich vor dem zu überwachenden Bereich 11 mit einem zweiten Gassensor 30 erfolgen, der in dem hier dargestellten Ausführungsbeispiel ebenfalls als Leitfähigkeitssensor ausgeführt ist. Ist die Leitfähigkeit an dem Gassensor 20 nach Durchströmen des zu überwachenden Bereiches 11 kleiner als die Leitfähigkeit der luftblasenfreien Flüssigkeit, die mittels des Gassensors 30 gemessen wird, so deutet dies auf einen Lufteintrag hin. Aus der Verminderung der Leitfähigkeit gegenüber dem Referenzwert kann die Menge der über die Leckstelle 12 eintretenden Luft abgeschätzt werden.

Figur 3 zeigt eine Anordnung, die beispielsweise dann Anwendung finden kann, wenn eine Referenzmessung nicht möglich ist. In diesem Fall kann eine Leckage durch Variation des Unterdruckes in dem zu überwachenden Bereich 11 erkannt werden. Gleiche oder funktionsgleiche Teile der Anordnung sind mit denselben Bezugszeichen versehen wie in Figur 1.

Mit dem Bezugzeichen 40 ist ein Druckregler gekennzeichnet, mittels dessen die Druckverhältnisse in dem zu überwachenden Bereich variierbar sind.

Bei einem Leck 12 in dem zu überwachenden Bereich 11 steigt die Eindringrate der Luft mit steigendem Druckgradienten zwischen Umgebung und dem Bereich 11. Die Förderstromerhöhung durch die Pumpe 60 führt zu einem verstärkten Eindringen von Luft durch Absenkung des Innendrucks in dem Bereich 11.

Da die Absenkung des Innendrucks in dem Bereich 11 durch Flusserhöhung einer nach dem Unterdruckbereich 11 liegenden Förderpumpe 60 proportional zur Förderstromerhöhung sein kann, ist in diesem Fall die Menge der durch die Leckstelle 12 einströmenden Luft zwar absolut größer als bei kleinerer Förderrate der Pumpe 60, jedoch kann der Anteil der einströmenden Luft im Verhältnis zum Gesamtstrom konstant bleiben, so dass eine Leitfähigkeitsänderung letztlich ausbleibt. Zu diesem Zwecke ist ein Druckregler 40 stromaufwärts des Bereichs 11 vorgesehen, der in Form einer regelbaren Pumpe oder eines regelbaren Ventils ausgeführt sein kann und mittels dessen eine Entkopplung zwischen Innendruck und Volumenstrom möglich ist.

Bei der Dialysierflüssigkeit kann es sich bei der vorliegenden Erfindung auch um reines Ultrafiltrat handeln, wenn z.B. bei den in Fig. 1 bis 3 gezeigten Anordnungen die Zuleitung geschlossen ist. Durch die vorliegende Erfindung kann selbstverständlich auch für diesen Fall eine Luftblasendetektion im Ultrafiltrat erfolgen, um die Dichtigkeit des entsprechenden Flüssigkeitskreislaufes zu überwachen.

Die vorliegende Erfindung lässt sich vorteilhaft zur Überwachung der Dichtigkeit der Teile eines Dialysierflüssigkeitskreislaufes verwenden, in dem wenigstens bereichsweise ein Unterdruck gegenüber der Umgebung vorliegt. Diese Unterdruckbereiche können sowohl Teile des Dialysierflüssigkeitskreislaufes innerhalb eines Dialysegerätes als auch externe Dialysierflüssigkeitskreisläufe mit einem Dialysator sein. Gerade hier kann es unter Umständen zu Undichtigkeiten bei der Konnektion kommen, die gegebenenfalls nicht vor Beginn der Behandlung erkannt werden können.

Der Begriff "Dialysierflüssigkeitskreislauf" gemäß der vorliegenden Erfindung ist weit auszulegen und kann neben Leitungen zur Führung von Dialysierflüssigkeit auch in den Leitungen befindliche Komponenten, wie den Dialysator, Pumpen, etc. sowie auch mit diesen in Verbindung stehende Einheiten, wie Steuergeräte, die genannte Auswerteeinheit etc. umfassen.

Auch der Begriff "Dialysierflüssigkeit" ist weit auszulegen: Dabei kann es sich um jede Flüssigkeit handeln, welche in einer Betriebsphase eines Dialysegeräts durch den Dialysierflüssigkeitskreislauf strömt, wie zum Beispiel zugegebenes Dialysat, reines Ultrafiltrat oder auch Reinigungsflüssigkeit, welche beim Reinigen durch den Dialysierflüssigkeitskreislauf strömt. Damit kann der Dialysierflüssigkeitskreislauf erfindungsgemäß in allen Betriebsphasen auf Undichtigkeiten überwacht werden.

**Patentansprüche**

1.  Dialysierflüssigkeitskreislauf (10) mit Leitungen zur Führung von Dialysierflüssigkeit, mit Mitteln zur Erkennung von Luft in der Dialysierflüssigkeit sowie mit einem in dem Dialysierflüssigkeitskreislauf (10) angeordneten Dialysator,
    **dadurch gekennzeichnet, dass** die Mittel wenigstens einen im Betrieb des Dialysierflüssigkeitskreislaufes kontinuierlich von der Dialysierflüssigkeit durchströmten Gassensor (20) umfassen, der derart ausgeführt ist, dass er wenigstens eine Eigenschaft der Dialysierflüssigkeit, die von der Anwesenheit von Luftblasen in der Dialysierflüssigkeit abhängt, misst und der stromabwärts eines zu überwachenden Bereiches (11) des Dialysierflüssigkeitskreislaufes (10), nämlich stromabwärts des in dem Dialysierflüssigkeitskreislauf befindlichen Dialysators angeordnet ist, in dem im Betrieb des Dialysierflüssigkeitskreislaufes (10) gegenüber Atmosphärendruck Unterdruck herrscht, wobei die Mittel ferner eine mit dem Gassensor (20) in Verbindung stehende Auswerteeinheit aufweisen, die derart ausgeführt ist, dass die mittels des Gassensors (20) gemessene Eigenschaft der Dialysierflüssigkeit im Hinblick auf das Vorhandensein von Luftblasen in der Dialysierflüssigkeit ausgewertet wird, wodurch der Dialysierflüssigkeitskreislauf auf Undichtigkeiten überwacht wird.

2.  Dialysierflüssigkeitskreislauf (10) nach Anspruch 1, **dadurch gekennzeichnet, dass** es sich bei dem Gassensor (20) um einen Leitfähigkeitssensor handelt.

3.  Dialysierflüssigkeitskreislauf (10) nach Anspruch 1 oder 2,
    **dadurch gekennzeichnet, dass** der Gassensor (20) derart ausgeführt ist, dass mittels des Gassensors (20) einzelne Signalspitzen der Eigenschaft der Dialysierflüssigkeit erfassbar sind.

4.  Dialysierflüssigkeitskreislauf (10) nach Anspruch 1 oder 2,
    **dadurch gekennzeichnet, dass** der Gassensor (20) und/oder die Auswerteeinheit derart ausgeführt ist, dass ein Mittelwert der wenigstens einen Eigenschaft der Dialysierflüssigkeit bestimmt wird und der Auswertung zugrunde gelegt wird.

5.  Dialysierflüssigkeitskreislauf (10) nach einem der vorhergehenden Ansprüche,
    **dadurch gekennzeichnet, dass** die Auswerteeinheit derart ausgeführt ist, dass sie ein Signal abgibt und/oder eine bestimmte Betriebsweise des Dialysierflüssigkeitskreislaufes (10) veranlasst, wenn bei der Auswertung festgestellt wird, dass Luftblasen in der Dialysierflüssigkeit vorliegen.

6.  Dialysierflüssigkeitskreislauf (10) nach Anspruch 5, **dadurch gekennzeichnet, dass** es sich bei der besonderen Betriebsweise um einen Druckhaltetest des Dialysierflüssigkeitskreislaufes (10) oder des zu überwachenden Bereichs (11) handelt.

7.  Dialysierflüssigkeitskreislauf (10) nach einem der vorhergehenden Ansprüche,
    **dadurch gekennzeichnet, dass** eine Bypassleitung (14) vorgesehen ist, die den zu überwachenden Bereich umgeht und dass wenigstens ein Absperrventil (16) vorgesehen ist, in dessen geöffnetem Zustand die Durchströmung der Bypassleitung (14) mit Dialysierflüssigkeit zur Durchführung einer Referenzmessung möglich ist.

**8.** Dialysierflüssigkeitskreislauf (10) nach einem der vorhergehenden Ansprüche, **dadurch gekennzeichnet, dass** stromaufwärts des zu überwachenden Bereiches (11) des Dialysierflüssigkeitskreislaufes (10) ein weiterer Gassensor (30) zur Durchführung einer Referenzmessung angeordnet ist.

**9.** Dialysierflüssigkeitskreislauf (10) nach einem der vorhergehenden Ansprüche, **dadurch gekennzeichnet, dass** Mittel zur Veränderung des Druckes in dem zu überwachenden Bereich (11) vorgesehen sind.

**10.** Dialysierflüssigkeitskreislauf (10) nach Anspruch 9, **dadurch gekennzeichnet, dass** die Mittel durch einen stromaufwärts des zu überwachenden Bereichs (11) angeordneten Druckregler (40) und durch eine stromabwärts des zu überwachenden Bereichs (11) angeordnete Pumpe (60) gebildet werden.

**11.** Dialysierflüssigkeitskreislauf (10) nach Anspruch 10, **dadurch gekennzeichnet, dass** der Druckregler (40) durch ein regelbares Ventil oder durch eine regelbare Pumpe gebildet wird.

**12.** Dialysierflüssigkeitskreislauf (10) pach einem der vorhergehenden Ansprüche, **dadurch gekennzeichnet, dass** in dem Dialysierflüssigkeitskreislauf (10) wenigstens ein Drucksensor zur Erfassung des in der Dialysierflüssigkeit herrschenden Drucks angeordnet ist.

**13.** Verfahren zur Erkennung von Luft in einer einen Dialysierflüssigkeitskreislauf (10) durchströmenden Dialysierflüssigkeit, wobei in dem Dialysierflüssigkeitskreislauf ein Dialysator angeordnet ist, **dadurch gekennzeichnet, dass** stromabwärts eines zu überwachenden Bereiches (11) des Dialysierflüssigkeitskreislaufes (10), in dem der Dialysator angeordnet ist und in dem im Betrieb des Dialysierflüssigkeitskreislaufes (10) gegenüber Atmosphärendruck Unterdruck herrscht, im Betrieb des Dialysierflüssigkeitskreislaufes kontinuierlich eine Eigenschaft der einen Messbereich durchströmenden Dialysierflüssigkeit gemessen wird, die von der Anwesenheit von Luftblasen in der Dialysierflüssigkeit abhängt, und bei dem die gemessene Eigenschaft im Hinblick auf das Vorhandensein von Luftblasen in der Dialysierflüssigkeit und darauf basierend auf das Vorhandensein einer Undichtigkeit des Dialysierflüssigkeitskreislaufes (10) ausgewertet wird.

**14.** Verfahren nach Anspruch 13, **dadurch gekennzeichnet, dass** es sich bei der gemessenen Eigenschaft um die Leitfähigkeit der Dialysierflüssigkeit handelt.

**15.** Verfahren nach Anspruch 13 oder 14, **dadurch gekennzeichnet, dass** einzelne Signalspitzen der Eigenschaft der Dialysierflüssigkeit gemessen werden und dass auf der Grundlage dieser Messung die Auswertung erfolgt.

**16.** Verfahren nach Anspruch 13 oder 14, **dadurch gekennzeichnet, dass** ein Mittelwert der Eigenschaft der Dialysierflüssigkeit ermittelt wird und dass auf der Grundlage des Mittelwertes die Auswertung erfolgt.

**17.** Verfahren nach einem der Ansprüche 13 bis 16, **dadurch gekennzeichnet, dass** ein Signal abgegeben und/oder eine bestimmte Betriebsweise des Dialysierflüssigkeitskreislaufes (10) veranlasst wird, wenn bei der Auswertung des Signals des Gassensors (20) festgestellt wird, dass Luftblasen in der Dialysierflüssigkeit vorliegen.

**18.** Verfahren nach Anspruch 17, **dadurch gekennzeichnet, dass** es sich bei der besonderen Betriebsweise um einen Druckhaltetest des Dialysierflüssigkeitskreislaufes (10) handelt.

**19.** Verfahren nach einem der Ansprüche 13 bis 18, **dadurch gekennzeichnet, dass** wenigstens eine Referenzmessung durchgeführt wird, bei der die Eigenschaft der luftblasenfreien Dialysierflüssigkeit erfasst wird.

**20.** Verfahren nach Anspruch 19, **dadurch gekennzeichnet, dass** die Referenzmessung derart vorgenommen wird, dass Dialysierflüssigkeit im Bypass (14) um den zu überwachenden Bereich (11) geführt wird.

**21.** Verfahren nach Anspruch 19 oder 20, **dadurch gekennzeichnet, dass** die Referenzmessung derart vorgenommen wird, dass die Eigenschaft der Dialysierflüssigkeit stromaufwärts des zu überwachenden Bereichs (11) gemessen wird.

**22.** Verfahren nach einem der Ansprüche 13 bis 21, **dadurch gekennzeichnet, dass** zum Zwecke der Prüfung des Vorhandenseins von Luftblasen in der Dialysierflüssigkeit der Druck in dem zu überwachenden Bereich (11) variiert wird.

**23.** Verfahren nach Anspruch 22, **dadurch gekennzeichnet, dass** die Druckänderung bei nicht veränderter Strömungsrate der Dialysierflüssigkeit durch den zu überwachenden Bereich (11) vorgenommen wird.

**24.** Verwendung eines Gassensors (20) in einem Dialysierflüssigkeitskreislauf (10), wobei in dem Dialysierflüssigkeitskreislauf (10) ein Dialysator angeordnet ist,
**dadurch gekennzeichnet, dass** der Gassensor (20) zur Erkennung von Luftblasen in einer den Dialysierflüssigkeitskreislauf (10) durchströmenden Dialysierflüssigkeit dient und derart ausgeführt ist, dass er wenigstens eine Eigenschaft der Dialysierflüssigkeit misst, die von der Anwesenheit von Luftblasen in der Dialysierflüssigkeit abhängt, wobei der Gassensor (20) stromabwärts eines zu überwachenden Bereiches (11) des Dialysierflüssigkeitskreislaufes (10) angeordnet ist, in dem der Dialysator angeordnet ist und in dem im Betrieb des Dialysierflüssigkeitskreislaufes (10) gegenüber Atmosphärendruck Unterdruck herrscht, und kontinuierlich von der Dialysierflüssigkeit im Betrieb des Dialysierflüssigkeitskreislaufes durchströmt wird.

25. Verwendung nach Anspruch 24,
**dadurch gekennzeichnet, dass** es sich bei dem Gassensor (20) um einen Leitfähigkeitssensor handelt.

26. Verwendung nach Anspruch 24 oder 25,
**dadurch gekennzeichnet, dass** die mittels des Gassensors (20) gemessene Eigenschaft dahingehend ausgewertet wird, ob Luftblasen in der Dialysierflüssigkeit vorliegen.

27. Verwendung nach einem der Ansprüche 24 bis 26,
**dadurch gekennzeichnet, dass** mittels des Gassensors (20) Signalspitzen der Eigenschaft gemessen werden oder dass mittels des Gassensors oder auf der Grundlage der mittels des Gassensors gemessenen Eigenschaft ein Mittelwert der Eigenschaft gebildet wird.

28. Verwendung nach Anspruch 26 oder 27,
**dadurch gekennzeichnet, dass** ein Signal abgegeben und/oder eine bestimmte Betriebsweise des Dialysierflüssigkeitskreislaufes (10) veranlasst wird, wenn bei der Auswertung festgestellt wird, dass Luftblasen in der Dialysierflüssigkeit vorliegen.

29. Verwendung nach Anspruch 28,
**dadurch gekennzeichnet, dass** es sich bei der besonderen Betriebsweise um einen Druckhaltetest des Dialysierflüssigkeitskreislaufes (10) handelt.

30. Verwendung nach einem der Ansprüche 24 bis 29,
**dadurch gekennzeichnet, dass** wenigstens eine Referenzmessung durchgeführt wird, bei der die Eigenschaft der luftblasenfreien Dialysierflüssigkeit erfasst wird.

31. Verwendung nach Anspruch 30,
**dadurch gekennzeichnet, dass** die Referenzmessung derart vorgenommen wird, dass die luftblasenfreie Dialysierflüssigkeit im Bypass (14) um den zu überwachenden Bereich (11) geführt wird.

32. Verwendung nach Anspruch 30 oder 31,
**dadurch gekennzeichnet, dass** die Referenzmessung derart vorgenommen wird, dass die Eigenschaft der luftblasenfreien Dialysierflüssigkeit mittels eines weiteren Gassensors (30) stromaufwärts des zu überwachenden Bereichs (11) gemessen wird.

33. Verwendung nach einem der Ansprüche 24 bis 32,
**dadurch gekennzeichnet, dass** zum Zwecke der Prüfung des Vorhandenseins von Luftblasen in der Dialysierflüssigkeit der Druck in dem zu überwachenden Bereich (11) variiert wird.

34. Verwendung nach Anspruch 33,
**dadurch gekennzeichnet, dass** die Druckänderung bei nicht veränderter Strömungsrate der Dialysierflüssigkeit vorgenommen wird.

35. Dialysegerät mit wenigstens einem Dialysierflüssigkeitskreislauf (10) gemäß einem der Ansprüche 1 bis 12.

**Claims**

1. A dialysis liquid circuit (10) with conduits for conducting dialysis liquid, with means for detecting air in the dialysis liquid, and with a dialyser arranged in the dialysis liquid circuit (10),
**characterized in that** the means comprise at least one gas sensor (20) traversed continuously by the dialysis liquid in operation of the dialysis liquid circuit, which is configured such that it measures at least one property of the dialysis liquid which depends on the presence of air bubbles in the dialysis liquid, and which is arranged downstream of a region (11) to be monitored of the dialysis liquid circuit (10), namely downstream of the dialyser present in the dialysis liquid circuit (10), in which in operation of the dialysis liquid circuit (10) a negative pressure exists with respect to atmospheric pressure, wherein the means furthermore include an evaluation unit connected with the gas sensor (20), which is configured such that the property of the dialysis liquid measured by means of the gas sensor (20) is evaluated with regard to the presence of air bubbles in the dialysis liquid, whereby the dialysis liquid circuit is monitored for leaks.

2. The dialysis liquid circuit (10) according to claim 1,
**characterized in that** the gas sensor (20) is a conductivity sensor.

3. The dialysis liquid circuit (10) according to claim 1 or 2,
**characterized in that** the gas sensor (20) is configured such that by means of the gas sensor (20) individual signal peaks of the property of the dialysis liquid can be detected.

4. The dialysis liquid circuit (10) according to claim 1 or 2,
**characterized in that** the gas sensor (20) and/or the evaluation unit is configured such that a mean value of the at least one property of the dialysis liquid is determined and taken as a basis for the evaluation.

5. The dialysis liquid circuit (10) according to any one of the preceding claims,
**characterized in that** the evaluation unit is configured such that it emits a signal and/or initiates a particular mode of operation of the dialysis liquid circuit (10) when it is detected during the evaluation that air bubbles are present in the dialysis liquid.

6. The dialysis liquid circuit (10) according to claim 5,
**characterized in that** the special mode of operation is a pressure holding test of the dialysis liquid circuit (10) or of the region (11) to be monitored.

7. The dialysis liquid circuit (10) according to any one of the preceding claims,
**characterized in that** a bypass conduit (14) is provided, which bypasses the region to be monitored, and that at least one shut-off valve (16) is provided, in whose open condition dialysis liquid can flow through the bypass conduit (14) for performing a reference measurement.

8. The dialysis liquid circuit (10) according to any one of the preceding claims,
**characterized in that** upstream of the region (11) to be monitored of the dialysis liquid circuit (10) a further gas sensor (30) is arranged for performing a reference measurement.

9. The dialysis liquid circuit (10) according to any one of the preceding claims,
**characterized in that** means are provided for varying the pressure in the region (11) to be monitored.

10. The dialysis liquid circuit (10) according to claim 9,
**characterized in that** the means are formed by a pressure regulator (40) arranged upstream of the region (11) to be monitored and by a pump (60) arranged downstream of the region (11) to be monitored.

11. The dialysis liquid circuit (10) according to claim 10,
**characterized in that** the pressure regulator (40) is formed by an adjustable valve or by an adjustable pump.

12. The dialysis liquid circuit (10) according to any one of the preceding claims,
**characterized in that** in the dialysis liquid circuit (10) at least one pressure sensor is arranged for detecting the pressure existing in the dialysis liquid.

13. A method for detecting air in a dialysis liquid flowing through a dialysis liquid circuit (10), wherein a dialyser is arranged in the dialysis liquid circuit,
**characterized in that** downstream of a region (11) to be monitored of the dialysis liquid circuit (10), in which the dialyser is arranged and in which in operation of the dialysis liquid circuit (10) a negative pressure exists with respect to atmospheric pressure, in operation of the dialysis liquid circuit a property of the dialysis liquid flowing through a measurement region is measured continuously, which depends on the presence of air bubbles in the dialysis liquid, and in which the property measured is evaluated with regard to the presence of air bubbles in the dialysis liquid and based thereon the presence of a leak of the dialysis liquid circuit (10) is evaluated.

14. The method according to claim 13,
**characterized in that** the property measured is the conductivity of the dialysis liquid.

15. The method according to claim 13 or 14,
**characterized in that** individual signal peaks of the property of the dialysis liquid are measured and that the evaluation is made on the basis of this measurement.

16. The method according to claim 13 or 14,
**characterized in that** a mean value of the property of the dialysis liquid is determined and that the evaluation is made on the basis of the mean value.

17. The method according to any one of claims 13 to 16,
**characterized in that** a signal is emitted and/or a particular mode of operation of the dialysis liquid circuit (10) is initiated when it is detected during the evaluation of the signal of the gas sensor (20) that air bubbles are present in the dialysis liquid.

18. The method according to claim 17,
**characterized in that** the special mode of operation is a pressure holding test of the dialysis liquid circuit (10).

19. The method according to any one of claims 13 to 18,
**characterized in that** at least one reference measurement is performed, in which the property of the dialysis liquid free from air bubbles is detected.

20. The method according to claim 19,

**characterized in that** the reference measurement is performed such that dialysis liquid is guided in a bypass (14) around the region (11) to be monitored.

21. The method according to claim 19 or 20, **characterized in that** the reference measurement is performed such that the property of the dialysis liquid is measured upstream of the region (11) to be monitored.

22. The method according to any one of claims 13 to 21, **characterized in that** for the purpose of checking the presence of air bubbles in the dialysis liquid the pressure in the region (11) to be monitored is varied.

23. The method according to claim 22, **characterized in that** the change in pressure is made with an unchanged flow rate of the dialysis liquid through the region (11) to be monitored.

24. Use of a gas sensor (20) in a dialysis liquid circuit (10), wherein a dialyser is arranged in the dialysis liquid circuit (10), **characterized in that** the gas sensor (20) serves to detect air bubbles in a dialysis liquid flowing through the dialysis liquid circuit (10) and is configured such that it measures at least one property of the dialysis liquid which depends on the presence of air bubbles in the dialysis liquid, wherein the gas sensor (20) is arranged downstream of a region (11) to be monitored of the dialysis liquid circuit (10), in which the dialyser is arranged and in which in operation of the dialysis liquid circuit (10) a negative pressure exists with respect to atmospheric pressure, and is traversed continuously by the dialysis liquid in operation of the dialysis liquid circuit.

25. The use according to claim 24, **characterized in that** the gas sensor (20) is a conductivity sensor.

26. The use according to claim 24 or 25, **characterized in that** the property measured by means of the gas sensor (20) is evaluated as to whether air bubbles are present in the dialysis liquid.

27. The use according to any one of claims 24 to 26, **characterized in that** by means of the gas sensor (20) signal peaks of the property are measured or that by means of the gas sensor or on the basis of the property measured by means of the gas sensor a mean value of the property is formed.

28. The use according to claim 26 or 27, **characterized in that** a signal is emitted and/or a particular mode of operation of the dialysis liquid circuit (10) is initiated, when it is detected during the evaluation that air bubbles are present in the dialysis liquid.

29. The use according to claim 28, **characterized in that** the special mode of operation is a pressure holding test of the dialysis liquid circuit (10).

30. The use according to any one of claims 24 to 29, **characterized in that** at least one reference measurement is performed, in which the property of the dialysis liquid free from air bubbles is detected.

31. The use according to claim 30, **characterized in that** the reference measurement is performed such that the dialysis liquid free from air bubbles is guided in a bypass (14) around the region (11) to be monitored.

32. The use according to claim 30 or 31, **characterized in that** the reference measurement is performed such that the property of the dialysis liquid free from air bubbles is measured by means of a further gas sensor (30) upstream of the region (11) to be monitored.

33. The use according to any one of claims 24 to 32, **characterized in that** for the purpose of checking the presence of air bubbles in the dialysis liquid the pressure in the region (11) to be monitored is varied.

34. The use according to claim 33, **characterized in that** the change in pressure is performed with an unchanged flow rate of the dialysis liquid.

35. A dialysis apparatus with at least one dialysis liquid circuit (10) according to any one of claims 1 to 12.

**Revendications**

1. Circuit de liquide pour dialyse (10) avec des conduits destinés à guider le liquide pour dialyse, avec des moyens destinés à la détection d'air dans le liquide pour dialyse ainsi qu'avec un dialyseur disposé dans le circuit de liquide pour dialyse (10), **caractérisé en ce que** les moyens comprennent au moins un capteur de gaz (20) traversé en continu par le liquide pour dialyse pendant le fonctionnement du circuit de liquide pour dialyse, qui est réalisé de telle manière qu'il mesure au moins une propriété du liquide pour dialyse, qui dépend de la présence de bulles d'air dans le liquide pour dialyse, et qu'il est disposé en aval d'une zone à surveiller (11) du circuit de liquide pour dialyse (10), à savoir en aval du dialyseur se trouvant dans le circuit de liquide pour dialyse, dans laquelle une dépression règne pendant le fonctionnement du circuit de liquide pour dialyse (10) par rapport à la pression atmosphérique, les moyens présentant par ailleurs une unité d'analyse se trouvant en relation avec le capteur de gaz (20), qui est réalisée de sorte que la propriété du liquide pour dialyse mesurée à l'aide du capteur de gaz (20) est analysée par rapport à la présence de bulles d'air dans le liquide pour dialyse, par quoi le circuit de liquide pour dialyse est surveiller pour repérer des défauts d'étanchéité.

2. Circuit de liquide pour dialyse (10) selon la revendication 1,
**caractérisé en ce que** dans le cas du capteur de gaz (20), il s'agit d'un capteur de conductibilité.

3. Circuit de liquide pour dialyse (10) selon les revendications 1 ou 2,
**caractérisé en ce que** le capteur de gaz (20) est réalisé de sorte qu'à l'aide du capteur de gaz (20), des pics de signaux individuels de la propriété du liquide pour dialyse sont saisissables.

4. Circuit de liquide pour dialyse (10) selon les revendications 1 ou 2,
**caractérisé en ce que** le capteur de gaz (20) et/ou l'unité d'analyse sont réalisés de sorte qu'une valeur moyenne d'au moins une propriété du liquide pour dialyse est déterminée et que l'analyse est prise pour base de celle-ci.

5. Circuit de liquide pour dialyse (10) selon une quelconque des revendications précédentes,
**caractérisé en ce que** l'unité d'analyse est réalisée de sorte qu'elle émet un signal et/ou déclenche un mode de fonctionnement déterminé du circuit de liquide pour dialyse (10) lorsqu'il est constaté lors de l'analyse que des bulles d'air sont présentes dans le liquide pour dialyse.

6. Circuit de liquide pour dialyse (10) selon la revendication 5,
**caractérisé en ce que** dans le cas du mode de fonctionnement particulier, il s'agit d'un test de maintien de la pression du circuit de liquide pour dialyse (10) ou de la zone à surveiller (11).

7. Circuit de liquide pour dialyse (10) selon une quelconque des revendications précédentes,
**caractérisé en ce qu'**une conduite de dérivation (14) est prévue, qui contourne la zone à surveiller et **en ce qu'**au moins une soupape d'arrêt (16) est prévue, à l'état ouvert de laquelle la traversée de la conduite de dérivation (14) par du liquide pour dialyse est possible pour effectuer une mesure de référence.

8. Circuit de liquide pour dialyse (10) selon une quelconque des revendications précédentes,
**caractérisé en ce qu'**en amont de la zone à surveiller (11) du circuit de liquide pour dialyse (10), un autre capteur de gaz (30) est disposé pour effectuer une mesure de référence.

9. Circuit de liquide pour dialyse (10) selon une quelconque des revendications précédentes,
**caractérisé en ce que** des moyens sont prévus pour modifier la pression dans la zone à surveiller (11).

10. Circuit de liquide pour dialyse (10) selon la revendication 9,
**caractérisé en ce que** les moyens sont constitués par un régulateur de pression (40) disposé en amont de la zone à surveiller (11) et par une pompe (60) disposée en aval de la zone à surveiller (11).

11. Circuit de liquide pour dialyse (10) selon la revendication 10,
**caractérisé en ce que** le régulateur de pression (40) est constitué par une soupape réglable ou par une pompe réglable.

12. Circuit de liquide pour dialyse (10) selon une quelconque des revendications précédentes,
**caractérisé en ce que** dans le circuit de liquide pour dialyse (10), au moins un capteur de pression est disposé pour la saisie de la pression régnant dans le liquide pour dialyse.

13. Procédé destiné à la détection d'air dans un liquide pour dialyse traversant un circuit de liquide pour dialyse (10), un dialyseur étant disposé dans le circuit de liquide pour dialyse,
**caractérisé en ce qu'**en aval d'une zone à surveiller (11) du circuit de liquide pour dialyse (10), dans laquelle le dialyseur est disposé et dans celle dans laquelle règne une dépression par rapport à la pression atmosphérique pendant le fonctionnement du circuit de liquide pour dialyse (10), une propriété du liquide pour dialyse traversant une zone de mesure est mesurée pendant le fonctionnement du circuit de liquide pour dialyse, qui dépend de la présence de bulles d'air dans le liquide pour dialyse, et pour quoi la propriété mesurée est analysée par rapport à la présence de bulles d'air dans le liquide pour dialyse et en se basant sur celle-ci, par rapport à la présence d'un défaut d'étanchéité du circuit de liquide pour dialyse (10).

14. Procédé selon la revendication 13,
**caractérisé en ce que** dans le cas de la propriété mesurée, il s'agit de la conductibilité du liquide pour dialyse.

15. Procédé selon les revendications 13 ou 14,
**caractérisé en ce que** des pics de signaux individuels de la propriété du liquide pour dialyse sont mesurés et **en ce que** sur la base de cette mesure, l'analyse est réalisée.

**16.** Procédé selon les revendications 13 ou 14, **caractérisé en ce qu'**une valeur moyenne de la propriété du liquide pour dialyse est analysée et **en ce que** sur la base de la valeur moyenne, l'analyse est réalisée.

**17.** Procédé selon une quelconque des revendications 13 à 16, **caractérisé en ce qu'**un signal est émis et/ou un mode de fonctionnement déterminé du circuit de liquide pour dialyse (10) est déclenché, lorsqu'il est constaté lors de l'analyse du signal du capteur de gaz (20), que des bulles d'air sont présentes dans le liquide pour dialyse.

**18.** Procédé selon la revendication 17, **caractérisé en ce que** dans le cas du mode de fonctionnement particulier, il s'agit d'un test de maintien de la pression du circuit de liquide pour dialyse (10).

**19.** Procédé selon une quelconque des revendications 13 à 18, **caractérisé en ce qu'**au moins une mesure de référence est effectuée, lors de laquelle la propriété du liquide pour dialyse sans bulles d'air est saisie.

**20.** Procédé selon la revendication 19, **caractérisé en ce que** la mesure de référence est réalisée de sorte que du liquide pour dialyse est conduit dans la dérivation (14) autour de la zone à surveiller (11).

**21.** Procédé selon les revendications 19 ou 20, **caractérisé en ce que** la mesure de référence est effectuée de sorte que les propriétés du liquide pour dialyse sont mesurées en amont de la zone à surveiller (11).

**22.** Procédé selon une quelconque des revendications 13 à 21, **caractérisé en ce que** dans l'objectif du contrôle de la présence de bulles d'air dans le liquide pour dialyse, la pression est variée dans la zone à surveiller (11).

**23.** Procédé selon la revendication 22, **caractérisé en ce que** la modification de pression est effectuée par la zone à surveiller (11) en cas de taux d'écoulement non modifié du liquide pour dialyse.

**24.** Utilisation d'un capteur de gaz (20) dans un circuit de liquide pour dialyse (10), un dialyseur étant disposé dans le circuit de liquide pour dialyse (10), **caractérisée en ce que** le capteur de gaz (20) sert à la détection de bulles d'air dans un liquide pour dialyse traversant le circuit de liquide pour dialyse (10) et est exécuté de sorte qu'il mesure au moins une propriété du liquide pour dialyse, qui dépend de la présence de bulles d'air dans le liquide pour dialyse, le capteur de gaz (20) étant disposé en amont d'une zone à surveiller (11) du circuit de liquide pour dialyse (10), dans laquelle le dialyseur est disposé et dans laquelle une dépression règne pendant le fonctionnement du circuit de liquide pour dialyse (10) par rapport à la pression atmosphérique, et étant traversé en continu par le liquide pour dialyse pendant le fonctionnement du circuit de liquide pour dialyse.

**25.** Utilisation selon la revendication 24, **caractérisée en ce que** dans le cas du capteur de gaz (20), il s'agit d'un capteur de conductibilité.

**26.** Utilisation selon les revendications 24 ou 25, **caractérisée en ce que** la propriété mesurée à l'aide du capteur de gaz (20) est évaluée dans l'objectif de constater si des bulles d'air sont présentes dans le liquide pour dialyse.

**27.** Utilisation selon une quelconque des revendications 24 à 26, **caractérisée en ce qu'**à l'aide du capteur de gaz (20), des pics de signaux de la propriété du liquide pour dialyse sont mesurés ou **en ce que**, à l'aide du capteur de gaz ou sur la base de la propriété mesurée à l'aide du capteur de gaz, une valeur moyenne de la propriété est constituée.

**28.** Utilisation selon les revendications 26 ou 27, **caractérisée en ce qu'**un signal est émis et/ou un mode de fonctionnement déterminé du circuit de liquide pour dialyse (10) est déclenché, lorsqu'il est constaté lors de l'analyse que des bulles d'air sont présentes dans le liquide pour dialyse.

**29.** Utilisation selon la revendication 28, **caractérisée en ce que** dans le cas du mode de fonctionnement particulier, il s'agit d'un test de maintien de la pression du circuit de liquide pour dialyse (10).

**30.** Utilisation selon une quelconque des revendications 24 à 29, **caractérisée en ce qu'**au moins une mesure de référence est effectuée, lors de laquelle la propriété du liquide pour dialyse sans bulles d'air est saisie.

**31.** Utilisation selon la revendication 30, **caractérisée en ce que** la mesure de référence est réalisée de sorte que le liquide pour dialyse sans bulle d'air est conduit dans la dérivation (14) autour de la zone à surveiller (11).

**32.** Utilisation selon les revendications 30 ou 31, **caractérisée en ce que** la mesure de référence est effectuée de sorte que les propriétés du liquide pour dialyse sans bulles d'air sont mesurées en amont de la zone à surveiller (11) au moyen d'un autre capteur à gaz (30).

**33.** Utilisation selon une quelconque des revendications 24 à 32, **caractérisée en ce que** dans l'objectif du contrôle de la présence de bulles d'air dans le liquide pour dialyse, la pression est variée dans la zone à surveiller (11).

**34.** Utilisation selon la revendication 33, **caractérisée en ce que** la modification de pression n'est pas effectuée en cas de taux d'écoulement non modifié du liquide pour dialyse.

**35.** Appareil de dialyse avec au moins un circuit de liquide pour dialyse (10) selon une quelconque des revendications 1 à 12.

FIG. 1

FIG. 2

**FIG. 3**

**FIG. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9604401 A
- US 5783072 A
- DE 19651355 A1